# EUROPEAN PATENT SPECIFICATION

(11) **EP 3 501 595 B1**
(45) Date of publication and mention of the grant of the patent: **29.07.2020**
(21) Application number: 18215784.2
(22) Date of filing: 21.12.2018
(51) Int. Cl.: A61N 1/32, A61N 1/04, A61B 18/14, A61B 18/00

(54) **HANDPIECE HAVING OF EXPANDABLE ELECTRODES USED FOR ELECTROPORATION**
HANDSTÜCK MIT ERWEITERBAREN ELEKTRODEN FÜR DIE ELEKTROPORATION
PIÈCE À MAIN COMPRENANT DES ÉLECTRODES EXPANSIBLES UTILISÉES POUR L'ÉLECTROPORATION

(30) Priority: 22.12.2017 IT 201700149360
(43) Date of publication of application: 26.06.2019
(73) Proprietor: IGEA S.p.A., 41012 Carpi (IT)
(72) Inventor: CADOSSI, Ruggero, 41012 CARPI (MO) (IT); MARAZZI, Donata, 41012 CARPI (MO) (IT); CADOSSI, Matteo, 41012 CARPI (MO) (IT); PERAZZOLO GALLO, Giacomo, 41012 CARPI (MO) (IT)
(74) Representative: Bongiovanni, Simone

(56) References cited:
- EP-A1- 2 032 057
- EP-A1- 2 693 959
- US-A1- 2012 150 172
- US-A1- 2014 081 255

## Description

### TECHNICAL FIELD

The present invention concerns a handpiece provided with expandable electrodes for use in an electroporation procedure.

### BACKGROUND ART

As is known, the technique of electroporation of a tissue is designed to uniformly expose all the cells contained in the tissue to an electric field having intensity higher than a local threshold value in order to obtain a permeabilization effect on the cell membranes. In some applications (for example ablation of tumour tissues) said local threshold value is of the order of 400V/cm (in general for values higher than 250V).

The tissue to be subjected to electroporation must be uniformly exposed to the electric field which must have a value higher than the threshold throughout the volume of application of the electric field.

In order to make said electric field spatially effective, in many therapeutic applications, pairs of needle-shaped electrodes are used, which are inserted in the tissue to be treated at the same depth, maintaining the parallelism between the needles. Fixed geometry electrodes can be used, for example, or provided with pairs of electrodes rigidly fixed to each other in order to guarantee the parallelism between the needles.

The requirements for parallelism between the electrodes and penetration uniformity are not easily met when treating tumour nodules positioned in locations that are not easy to access such as hollow or visceral organs. This requirement is difficult to apply if open surgery is not desired, for example in surgical procedures on hepato-biliary-pancreatic tumours.

To treat the above-mentioned tumour sites, ablation techniques have been proposed (for example radiofrequency) in which a handpiece is used provided with an expandable band of electrodes shaped in the form of an elastic metal cable provided with shaped end portions so as to be used like a needle.

The needle end portions are inserted in the tumour nodule and the electrodes are subsequently positioned and/or expanded in the tissue. The electromagnetic energy released by the electrodes produces significant heating of the surrounding tissue causing degenerative coagulation of said tissue.

An example of handpiece provided with expandable electrodes of the type described above is described in the patent EP-B-2.032.057 which illustrates a support assembly comprising an insulating body elongated along an axis and internally defining a plurality of internal channels, each of which develops from a first distal end of the elongated body along a rectilinear segment parallel to the axis, contiguous to and communicating with a second curved segment which has a radial distance relative to the axis, increasing towards a second proximal end portion of the elongated body. Each curved segment joins with the second proximal end through a respective opening. The handpiece further comprises a plurality of flexible electrodes (needles) carried by the support assembly and movable relative to the body due to the action of a manual type thrust system; each electrode comprises an elastic metal cable made of conductive material covered by an insulating sheath and provided with an uncovered front portion that forms a needle-shaped active portion. Each elastic cable is housed inside a respective channel with the active portion which, in use, exits from the respective aperture. The manual thrust system acts on the rear portion of the flexible cables to produce a movement of said cables in a feeding direction in which the front portions of the cables that exit from the support assembly advance along the axis and, at the same time, bend and radially move farther away from said axis.

### DISCLOSURE OF INVENTION

The object of the present invention is to provide a handpiece of the above-mentioned type which is also provided with a flexible central electrode sliding inside a rectilinear channel which is coaxial with the axis and also joins with the second proximal end through a respective opening. The flexible central electrode is movable axially in opposite directions. According to the present invention, the movement of the flexible electrodes and the central electrode is produced independently.

The preceding object is achieved by the present invention as the latter concerns a handpiece provided with expandable electrodes used in electroporation procedures of the type described in claim 1.

### BRIEF DESCRIPTION OF THE DRAWINGS

The invention will now be illustrated with reference to the accompanying drawings which show a preferred non-limiting embodiment thereof in which:
- figure 1 illustrates a first side view of the handpiece produced according to the teachings of the present invention;
- figure 2 illustrates a second side view with partially shown internal parts of the handpiece produced according to the teachings of the present invention;
- figure 3 illustrates a front view of the handpiece produced according to the teachings of the present invention;
- figure 4 illustrates a cross-section of the handpiece according the section IV-IV of figure 3;
- figure 5 illustrates, on an enlarged scale and in section, a detail of the handpiece; and
- figure 6 illustrates a component element of the handpiece.

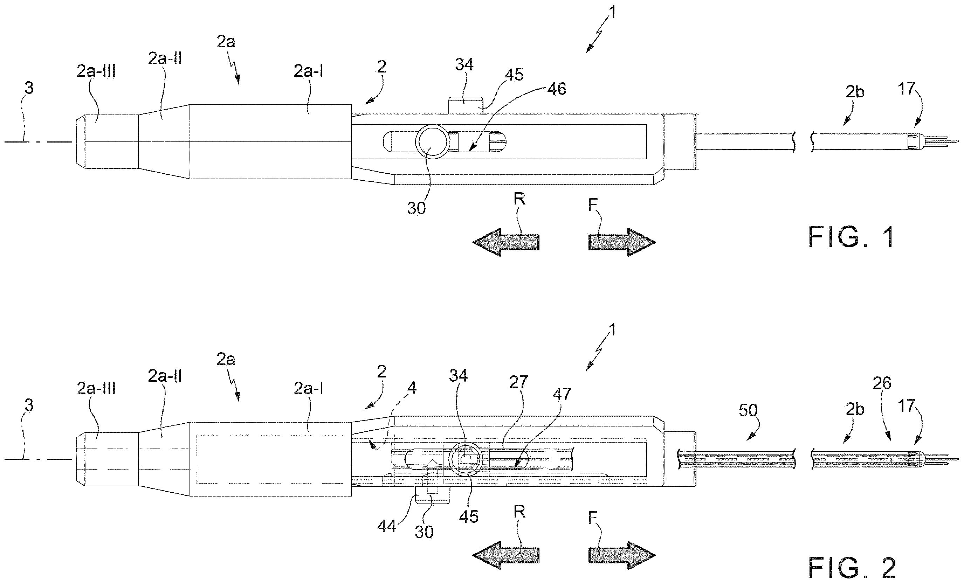

**In** **figure 1** **the number 1 denotes,** overall, a handpiece used in electroporation procedures comprising an electrode support body 2 of elongated shape along an axis 3 provided with a first end portion 2a configured to be gripped.

### BEST MODE FOR CARRYING OUT THE INVENTION

Expediently, according to the embodiment example illustrated, the first end portion 2a comprises a first cylindrical element 2a-I coaxial with the axis 3, a frustoconical element 2a-II integral with the first cylindrical element 2a-I and coaxial with the axis 3 and a second cylindrical end element 2a-III integral with the frustoconical element 2a-II, coaxial with the axis 3 and having smaller diameter than the diameter of the first cylindrical element 2a-I.

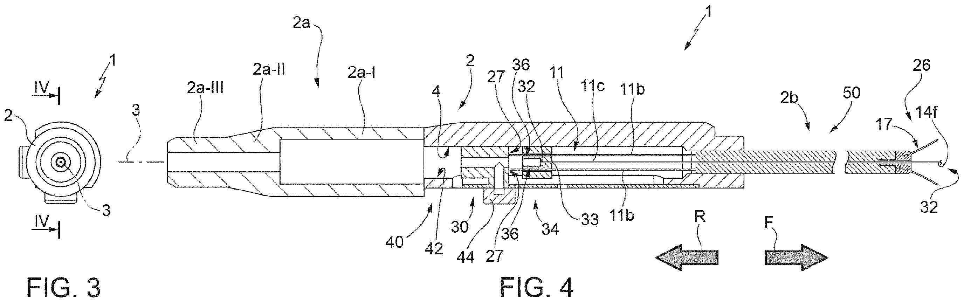

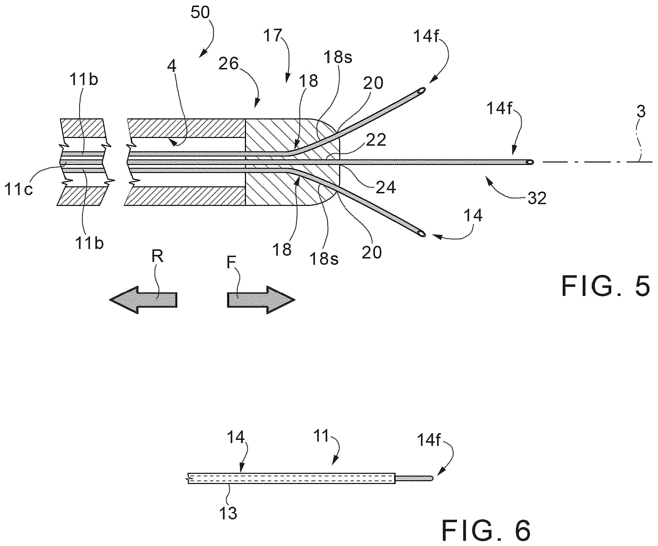

The support body 2 defines an elongated internal seat 4 along the axis 3 (see figures 2 and 4) and housing a plurality of flexible electrodes 11 (figure 4) each comprising an elastic cable 14 made of a conductive metal material covered by an insulating sheath 13 (figure 6). Each flexible electrode 11 is provided with an uncovered front portion 14-f which forms a needle-shaped active portion.

The handpiece 1 further comprises an electrode deflection assembly 17 arranged at a second end portion 2b of the support body 2 and provided, in the embodiment example, with first internal channels 18 (see the enlargement of figure 5) which communicate, on one side, with the elongated internal seat 4 and are provided with at least one end segment 18s which forms an angle of divergence **alpha** relative to the axis 3 having radial distance d1 relative to the axis 3 increasing towards a front end portion in which the channel 18 joins with the outside of the guide assembly 17 through a respective first opening 20. Alternatively, the channels 18 could be formed on bodies that extend externally to the deflection assembly 17 which would be provided inside with rectilinear channels.

**According to the present invention,** the electrode deflection assembly 17 comprises at least one second rectilinear internal channel 22 which communicates, on one side, with the elongated internal seat 4, extends coaxial with the axis 3 and joins with the outside of the guide assembly 17 through a respective second opening 24.

The flexible electrodes 11 comprise peripheral flexible electrodes 11-b provided with first end portions 26 (figures 4 and 5) which engage the first internal channels 18 exiting, in use, from the first openings 20; the peripheral flexible electrodes 11-b have second end portions 27 coupled with a first slider 30 sliding along the elongated seat 4. The movement of the first slider 30 in a first feeding direction (F) performs exiting of the front portions 14-f of the peripheral electrodes 11-b which exit from the electrode deflection assembly 17, advance along the axis 3 and, at the same time, bend, radially moving farther away from said axis 3. The movement of the first slider 30 sliding along a second return direction R opposite the first direction (F) performs re-entering of the front portions 14f of the electrodes 11-b inside the deflection assembly 17: during said return motion the front portions 14f radially approach said axis 3.

The flexible electrodes 11 comprise at least one flexible central electrode 11-c provided with a first end portion 32 which engages the rectilinear channel 22 exiting from the second opening 24. The central electrode 11-c has a second end portion 33 (figure 4) coupled with a second slider 34 sliding along the elongated seat 4.

The movement of the second slider 34 along the first feeding direction F performs exiting of the front portion 14f of the central electrode 11-c which exits from the guide assembly 17 without bending and advances along the axis 3. The movement of the second slider 34 along the second return direction (F) causes re-entry of the front portion 14f of the central electrode 11-c which retracts along the axis 3. Also in this case, the central electrode 11-c does not bend.

The first slider 30 is arranged inside the elongated seat 4 (see figures 2 and 4) between an end portion of the elongated seat 4 adjacent to the gripping end portion 2a and the second slider body 34 which is also housed in the elongated seat 4. The second end portions 27 of the peripheral electrodes 11-b are stably coupled to the first slider 30 and the peripheral electrodes 11/b cross the second slider 34 engaging through holes 36 which extend in an axial direction through the second slider 34. In turn, the second end portion 33 of the central electrode 11-c is stably coupled to the second slider 34.

The movement of the first slider 30 along the feeding direction brings the first slider 30 into abutment on the face of the second slider 34 facing the first slider 30 to perform, following said abutment, a synchronized feeding of the peripheral electrodes 11-b and of the central electrode 11-c.

In turn the movement of the second slider 34 along the retraction direction R brings the second slider 34 into abutment on the face of the first slider 30 facing the second slider 34 to perform, following said abutment, a synchronized retraction of the peripheral electrodes 11-b and of the central electrode 11-c.

In further detail, the elongated support body 2 comprises a portion 40 proximal to the gripping portion which internally defines a cylindrical chamber 42 (figure 4) which forms the part of the elongated seat 4 inside which the first and the second slider 30 and 34 slide; the first and the second slider 30, 34 comprise respective cylindrical pistons sliding in the cylindrical chamber 42 and radial appendages 44, 45 which engage first and second rectilinear grooves 46, 47 formed on the proximal portion 40. The radial appendages 44,45 are provided with end portions which protrude from the respective grooves 46,47 and are configured to be moved with the end of a finger, i.e. manipulated to allow the manual axial movement of the first and the second slider 30,34 and therefore move the peripheral electrodes 11-b/central electrode 11-c.

The first and the second rectilinear grooves 46,47 extend along planes substantially perpendicular to each other. This facilitates operation of the first and the second slider 30 and 34 by means of different fingers of the hand, in particular the thumb and index finger.

The elongated support body 2 further comprises a tubular element 50 which extends coaxial to the axis 3 from the portion 40 and defines an internal cavity which communicates with the cylindrical chamber 42 forming, together with the chamber 42, the elongated seat 4 for housing the flexible electrodes.

The electrode deflection assembly 17 is arranged at the free end of the tubular element 50.

The tubular element 50 which has a length of a few centimetres and reduced diameter (less than one cm) allows introduction of the deflection assembly 17 from which the electrodes protrude inside the cavity of the human body during surgical operations.

The second end portions 27 and 33 of the flexible electrodes 11 are electrically connected to electrical conductors (not illustrated) which extend through the gripping end portion 2a and are used to supply electrical signals used for electroporation processes.

In use, after resting the end of the tubular element 50 on the portion of tissue to be treated (the patient is under general or local anaesthetic), an operator moves the slider 34 in the direction F causing exit of the central electrode 11-c.

The position of the slider 34 along the groove 47 adjusts the penetration depth. For said purpose the groove 47 could be provided with a graduated scale.

The central electrode 11-c is inserted in the tissue in order to facilitate ultrasound viewing and fix the target tumour mass.

The operator then moves the slider 30 along the direction F performing the synchronized exit of all the peripheral electrodes 11-b. The position of the slider 30 along the groove 46 adjusts the penetration depth and width of the treated area. For said purpose the groove 46 could be provided with a graduated scale.

The electrodes 11-b and 11-c are then powered and an electroporation cycle is performed according to a known medical protocol. At the end of the treatment the electrodes 11-b and 11-c are extracted from the tissue.

According to a first retraction form, the operator moves the slider 30 along the direction R performing synchronized retraction of all the peripheral electrodes 11-b.

The operator then moves the slider 34 along the direction R, achieving the retraction of the central electrode 11-c.

At the end of said operation all the needles are extracted from the tissue.

If the operator performs a second form of retraction in which the slider 34 is initially moved to achieve the retraction of the central electrode 11-c, then all the peripheral electrodes 11-b are also retracted. As illustrated above, the slider 34 abuts on the slider 30 and moves it.

Also in this case, at the end of said operation all the needles are extracted from the tissue.

## Claims

1. A handpiece (1) for use in an electroporation procedure comprising:
- an electrode support body (2) having an elongated shape along an axis (3) provided with a first end portion (2a) configured to be gripped;
said electrode support body (2) defining an elongated internal seat (4) along said axis (3) and housing a plurality of flexible electrodes (11) each comprising an elastic cable (14) made of a conductive material covered by an insulating sheath (13) and provided with an uncovered front portion (14-f) which forms a needle-shaped active portion;
- an electrode deflection assembly (17) arranged at a second end portion (2b) of the electrode support body (2) and provided with first channels (18) which communicate, on one side, with the elongated internal seat (4) and are provided with at least one end segment (18s) which forms a divergence angle relative to the axis (3) having a radial distance relative to the axis (3) increasing towards a front end portion in which the channel (18) communicates with a respective first opening (20), **characterized in that** said electrode deflection assembly (17) comprises at least a second rectilinear channel (22) which communicates, on one side, with the elongated internal seat (4), extends coaxial with the axis (3) and joins with a respective second opening (24);
said flexible electrodes comprising peripheral flexible electrodes (11-b) provided with first end portions (26) which engage the first internal channels (18) exiting from the first openings (20); said peripheral flexible electrodes (11-b) having second end portions (27) coupled to a first slider (30) sliding along said elongated seat (4); the movement of the first slider (30) along a first feeding direction (F) performs the exiting of the front portions (14f) of the peripheral electrodes (11-b) that exit from the support assembly (17), advance along said axis (3) and, at the same time, radially move farther away from the axis (3) itself, the movement of the first slider (30) along a second return direction (F) performs the re-entering of the front portions (14-f) of the peripheral electrodes (11-b) which radially approach the axis (3) itself;
said flexible electrodes (11) comprising at least one central flexible electrode (11-c) provided with a first end portion (32) which engages the second rectilinear channel (22) coming out of the second opening (24); said central electrode having a second end portion (33) coupled to a second slider (34) sliding along said elongated seat (4); the movement of the second elongated slider (34) along the first feeding direction (F) performs the exiting of the front portion of the cable (14) from the deflection assembly (17) along said axis and the movement of the second elongated body along a second return direction (R) performs the re-entering of the front portion of the cables (14) towards the deflection assembly (17), wherein the first slider (30) is arranged inside the elongated seat (4) between an end portion of the elongated seat (4) adjacent to the first gripping end portion (2a) and the second slider body (34) which is also housed inside the elongated seat (4); said second end portions (27) of the peripheral electrodes (11-b) are stably coupled to the first slider (30) and the peripheral electrodes (11-b) pass through the second slider (34) engaging through holes (36) of the second slider (34); said second end portion (33) of the central electrode (11-c) is firmly coupled to the second slider (34); the movement of the first slider (30) along the feeding direction brings the first slider (30) into abutment on the face of the second slider (34) facing the first slider (30) to perform, following said abutment, a synchronized feeding of the peripheral electrodes (11-b) and of the central electrode (11-c); the movement of the second slider (34) along the retraction direction (R) brings the second slider (34) into abutment on the face of the first slider (30) facing the second slider (34) to perform, following said abutment, a synchronized retraction of the peripheral electrodes (11-b) and of the central electrode (11-c).

2. The handpiece according to claim 1, the elongated support body (2) comprises a portion (40) proximal to the gripping portion which internally defines a chamber (42) which forms the part of the elongated seat (4) inside which the first and the second slider (30 and 34) slide; the first and the second slider (30, 34) comprise respective pistons sliding inside the chamber (42) and radial appendages (44, 45) which engage first and second rectilinear grooves (46, 47) formed on the proximal portion (40); the radial appendages (44, 45) are provided with end portions protruding from the respective grooves (46, 47) and are configured to be handled so as to allow the manual axial movement of the first and the second sliders (30, 34).

3. The handpiece according to claim 2, wherein the first and the second rectilinear grooves (46, 47) extend along planes substantially perpendicular to each other.

4. The handpiece according to claim 2 or 3, wherein the elongated support body (2) further comprises a tubular element (50), which extends coaxial with the axis (3) from the proximal portion (40) and defines an internal cavity, which communicates with the cylindrical chamber (42) forming, together with the chamber (42), the elongated seat (4) for housing the flexible electrodes.

5. The handpiece according to claim 4, wherein said electrode deflection assembly (17) is arranged at the free end of the tubular element (50).

6. The handpiece according to claim 1, wherein the second end portions (27 and 33) of the flexible electrodes (11) are electrically connected to electrical conductors which extend through the gripping end portion (2a) and are used for supplying electrical signals used for the electroporation processes.

## Patentansprüche

1. Handstück (1) zur Verwendung bei einem Elektroporationsverfahren, das aufweist:
einen Elektrodenhaltekörper (2) mit einer länglichen Form entlang einer Achse (3), der mit einem ersten Endabschnitt (2a) versehen ist, welcher derart ausgebildet ist, dass er gegriffen wird;
wobei der Elektrodenhaltekörper (2) einen länglichen inneren Sitz (4) entlang der Achse (3) definiert und eine Vielzahl von flexiblen Elektroden (11) aufnimmt, die jeweils ein elastisches Kabel (14) aufweisen, das aus einem leitenden Material gefertigt ist und von einer Isolierummantelung (13) bedeckt ist und mit einem unbedeckten vorderen Abschnitt (14-f) versehen ist, der einen nadelartig geformten aktiven Abschnitt bildet;
eine Elektrodenauslenkanordnung (17), die an einem zweiten Endabschnitt (2b) des Elektrodenhaltekörpers (2) angeordnet ist und mit ersten Kanälen (18) versehen ist, die auf einer Seite mit dem länglichen inneren Sitz (4) kommunizieren und mit mindestens einem Endsegment (18s) versehen sind, das einen Divergenzwinkel relativ zu der Achse (3) mit einem radialen Abstand relativ zu der Achse (3) bildet, der sich in Richtung eines vorderen Endabschnitts, in dem der Kanal (18) mit einer jeweiligen ersten Öffnung (20) kommuniziert, vergrößert,
**dadurch gekennzeichnet, dass**
die Elektrodenauslenkanordnung (17) mindestens einen zweiten geradlinigen Kanal (22) aufweist, der auf einer Seite mit dem länglichen inneren Sitz (4) kommuniziert, sich koaxial mit der Achse (3) erstreckt und in eine jeweilige zweite Öffnung (24) eintritt;
wobei die flexiblen Elektroden periphere flexible Elektroden (11-b) aufweisen, die mit ersten Endabschnitten (26) versehen sind, welche mit den ersten inneren Kanälen (18) zusammengreifen, die aus den ersten Öffnungen (20) austreten; wobei die peripheren flexiblen Elektroden (11-b) zweite Endabschnitte (27) aufweisen, die mit einem ersten Schieber (30), der entlang des länglichen Sitzes (4) gleitet, verbunden sind; durch die Bewegung des ersten Schiebers (30) in einer ersten Zuführrichtung (F) das Austreten der vorderen Abschnitte (14f) der peripheren Elektroden (11-b) durchgeführt wird, die aus der Halteanordnung (17) austreten, entlang der Achse (3) vorgeschoben werden und sich gleichzeitig radial weiter von der Achse (3) selbst weg bewegen, durch die Bewegung des ersten Schiebers (30) in einer zweiten Rückkehrrichtung (R) das Wiedereintreten der vorderen Abschnitte (14-f) der peripheren Elektroden (11-b) durchgeführt wird, die sich der Achse (3) selbst radial nähern;
wobei die flexiblen Elektroden (11) mindestens eine zentrale flexible Elektrode (11-c) aufweisen, die mit einem ersten Endabschnitt (32) versehen ist, der mit dem zweiten geradlinigen Kanal (22) zusammengreift, welcher aus der zweiten Öffnung (24) heraustritt; wobei die zentrale Elektrode einen zweiten Endabschnitt (33) aufweist, der mit einem zweiten Schieber (34) verbunden ist, welcher entlang des länglichen Sitzes (4) gleitet; durch die Bewegung des zweiten länglichen Schiebers (34) in der ersten Zuführrichtung (F) das Austreten des vorderen Abschnitts des Kabels (24) aus der Auslenkanordnung (17) entlang der Achse durchgeführt wird und durch die Bewegung des zweiten länglichen Körpers in einer zweiten Rücckehrrichtung (R) das Wiedereintreten des vorderen Abschnitts der Kabel (14) in Richtung der Auslenkanordnung (17) durchgeführt wird, wobei der erste Schieber (30) in dem länglichen Sitz (4) zwischen einem Endabschnitt des länglichen Sitzes (4), der an den ersten Greif-Endabschnitt (2a) angrenzt, und dem zweiten Schieberkörper (34), der ebenfalls in dem länglichen Sitz (4) aufgenommen ist, angeordnet ist; die zweiten Endabschnitte (27) der peripheren Elektroden (11-b) stabil mit dem ersten Schieber (30) verbunden sind und die peripheren Elektroden (11-b) durch den zweiten Schieber (34) verlaufen und mit Durchgangslöchern (36) des zweiten Schiebers (34) zusammengreifen; der zweite Endabschnitt (33) der zentralen Elektrode (11-c) fest mit dem zweiten Schieber (34) verbunden ist; durch die Bewegung des ersten Schiebers (30) in der Zuführrichtung der erste Schieber (30) in Anlage an der Fläche des zweiten Schiebers (34), die dem ersten Schieber (30) zugewandt ist, gebracht wird, um im Anschluss an das Inanlagebringen eine synchronisierte Zuführung der peripheren Elektroden (11-b) und der zentralen Elektrode (11-c) durchzuführen; durch die Bewegung des zweiten Schiebers (34) in der Zurückziehrichtung (R) der zweite Schieber (34) in Anlage an der Fläche des ersten Schiebers (30), die dem zweiten Schieber (34) zugewandt ist, gebracht wird, um im Anschluss an das Inanlagebringen ein synchronisiertes Zurückziehen der peripheren Elektroden (11-b) und der zentralen Elektrode (11-c) durchzuführen.

2. Handstück nach Anspruch 1, bei dem der längliche Haltekörper (2) einen Abschnitt (40) proximal zu dem Griffabschnitt aufweist, der in seinem Inneren eine Kammer (42) definiert, die den Teil des länglichen Sitzes (4) bildet, in dem der erste und der zweite Schieber (30 und 34) gleiten; der erste und der zweite Schieber (30, 34) jeweilige Kolben, die in der Kammer (42) gleiten, und radiale Ansätze (44, 45) aufweisen, die mit ersten und zweiten geradlinigen Nuten (46, 47), welche an dem proximalen Abschnitt (40) ausgebildet sind, zusammengreifen; die radialen Ansätze (44, 45) mit Endabschnitten versehen sind, die von den jeweiligen Nuten (46, 47) vorstehen und derart ausgebildet sind, dass sie so gehandhabt werden, dass sie das manuelle axiale Bewegen des ersten und des zweiten Schiebers (30, 34) ermöglichen.

3. Handstück nach Anspruch 2, bei dem sich die erste und die zweite geradlinige Nut (46, 47) entlang Ebenen erstrecken, die im Wesentlichen rechtwinklig zueinander sind.

4. Handstück nach Anspruch 2 oder 3, bei dem der längliche Haltekörper (2) ferner ein rohrförmiges Element (50) aufweist, das sich koaxial mit der Achse (3) von dem proximalen Abschnitt (40) erstreckt und einen inneren Hohlraum definiert, der mit der zylindrischen Kammer (42) kommuniziert und zusammen mit der Kammer (42) den länglichen Sitz (4) zum Aufnehmen der flexiblen Elektroden bildet.

5. Handstück nach Anspruch 4, bei dem die Elektrodenauslenkanordnung (17) an dem freien Ende des rohrförmigen Elements (50) angeordnet ist.

6. Handstück nach Anspruch 1, bei dem die zweiten Endabschnitte (27 und 33) der flexiblen Elektroden (11) elektrisch mit elektrischen Leitern verbunden sind, die sich durch den Greif-Endabschnitt (2a) erstrecken und zum Liefern von elektrischen Signalen verwendet werden, die für die Elektroporationsprozesse verwendet werden.

## Revendications

1. Pièce à main (1) destinée à être utilisée dans une procédure d'électroporation comprenant :
un corps de support d'électrodes (2) ayant une forme allongée le long d'un axe (3) prévu avec une première partie d'extrémité (2a) configurée pour être saisie ;
ledit corps de support d'électrodes (2) définissant une base interne allongée (4) le long dudit axe (3) et logeant une pluralité d'électrodes flexibles (11) comprenant chacune un câble élastique (14) réalisé avec un matériau conducteur recouvert par une gaine isolante (13) et prévu avec une partie avant découverte (14-f) qui forme une partie active en forme d'aiguille ;
un ensemble de déflexion d'électrodes (17) agencé au niveau d'une seconde partie d'extrémité (2b) du corps de support d'électrodes (2) et prévu avec des premiers canaux (18) qui communiquent, d'un côté, avec la base interne allongée (4) et sont prévus avec au moins un segment d'extrémité (18s) qui forme un angle de divergence par rapport à l'axe (3) ayant une distance radiale par rapport à l'axe (3) augmentant vers une partie d'extrémité avant dans laquelle le canal (18) communique avec une première ouverture (20) respective,
**caractérisée en ce que** ledit ensemble de déflexion d'électrodes (17) comprend au moins un second canal rectiligne (22) qui communique, d'un côté, avec la base interne allongée (4), s'étend de manière coaxiale avec l'axe (3) et s'assemble avec une seconde ouverture (24) respective ;
lesdites électrodes flexibles comprenant des électrodes flexibles périphériques (11-b) prévues avec des premières parties d'extrémité (26) qui mettent en prise les premiers canaux internes (18) sortant des premières ouvertures (20) ; lesdites électrodes flexibles périphériques (11-b) ayant des secondes parties d'extrémité (27) couplées à une première glissière (30) coulissant le long de ladite base allongée (4) ; le mouvement de la première glissière (30) le long de la première direction d'alimentation (F) réalise la sortie des parties avant (14f) des électrodes périphériques (11-b) qui sortent de l'ensemble de support (17), avancent le long dudit axe (3) et, en même temps, se déplacent radialement plus à distance de l'axe (3) lui-même, le déplacement de la première glissière (30) le long d'une seconde direction de retour (F) réalise la nouvelle entrée des parties avant (14-f) des électrodes périphériques (11-b) qui se rapprochent axialement de l'axe (3) lui-même ;
lesdites électrodes flexibles (11) comprenant au moins une électrode flexible centrale (11-c) prévue avec une première partie d'extrémité (32) qui met en prise le second canal rectiligne (22) sortant de la seconde ouverture (24) ; ladite électrode centrale ayant une seconde partie d'extrémité (33) couplée à une seconde glissière (34) coulissant le long de ladite base allongée (4) ; le déplacement de la seconde glissière allongée (34) le long de la première direction d'alimentation (F) réalise la sortie de la partie avant du câble (14) de l'ensemble de déflexion (17) le long dudit axe et le déplacement du second corps allongé le long d'une seconde direction de retour (R) réalise la nouvelle entrée de la partie avant des câbles (14) vers l'ensemble de déflexion (17), dans laquelle la première glissière (30) est agencée à l'intérieur de la base allongés (4) entre une partie d'extrémité de la base allongée (4) adjacente à la première partie d'extrémité de préhension (2a) et le second corps de glissière (34) qui est également logé à l'intérieur de la base allongée (4) ; lesdites secondes parties d'extrémité (27) des électrodes périphériques (11-b) sont couplées de manière stable à la première glissière (30) et les électrodes périphériques (11-b) passent à travers la seconde glissière (34) qui met en prise des trous débouchants (36) de la seconde glissière (34) ; la seconde partie d'extrémité (33) de l'électrode centrale (11-c) est fermement couplée à la seconde glissière (34) ; le déplacement de la première glissière (30) le long de la direction d'alimentation amène la première glissière (30) en butée sur la face de la seconde glissière (34) faisant face à la première glissière (30) pour réaliser, suite à ladite butée, une alimentation synchronisée des électrodes périphériques (11-b) et de l'électrode centrale (11-c) ; le déplacement de la seconde glissière (34) le long de la direction de rétraction (R) amène la seconde glissière (34) en butée sur la face de la première glissière (30) faisant face à la seconde glissière (34) pour réaliser, suite à ladite butée, une rétraction synchronisée des électrodes périphériques (11-b) et de l'électrode centrale (11-c) .

2. Pièce à main selon la revendication 1, le corps de support allongé (2) comprend une partie (40) proximale par rapport à la partie de préhension qui définit intérieurement une chambre (42) qui forme la partie de la base allongée (4) à l'intérieur de laquelle la première et la seconde glissière (30 et 34) coulissent ; la première et la seconde glissière (30, 34) comprennent des pistons respectifs coulissant à l'intérieur de la chambre (42) et des appendices radiaux (44, 45) qui mettent en prise des première et seconde rainures rectilignes (46, 47) formées sur la partie proximale (40) ; les appendices radiaux (44, 45) sont prévus avec des parties d'extrémité dépassant des rainures (46, 47) respectives et sont configurés pour être manipulés afin de permettre le déplacement axial manuel de la première et de la seconde glissière (30, 34).

3. Pièce à main selon la revendication 2, dans laquelle la première et la seconde rainure rectiligne (46, 47) s'étendent le long de plans sensiblement perpendiculaires entre eux.

4. Pièce à main selon la revendication 2 ou 3, dans laquelle le corps de support allongé (2) comprend en outre un élément tubulaire (50) qui s'étend de manière coaxiale avec l'axe (3) à partir de la partie proximale (40) et définit une cavité interne qui communique avec la chambre cylindrique (42) formant, conjointement avec la chambre (42), la base allongée (4) pour loger les électrodes flexibles.

5. Pièce à main selon la revendication 4, dans laquelle ledit ensemble de déflexion d'électrodes (17) est agencé au niveau de l'extrémité libre de l'élément tubulaire (50).

6. Pièce à main selon la revendication 1, dans laquelle les secondes parties d'extrémité (27 et 33) des électrodes flexibles (11) sont électriquement raccordées aux conducteurs électriques qui s'étendent à travers la partie d'extrémité de préhension (2a) et sont utilisées pour fournir des signaux électriques utilisés pour les procédés d'électroporation.
